# EUROPEAN PATENT SPECIFICATION

(11) **EP 1 906 854 B1**
(45) Date of publication and mention of the grant of the patent: **08.04.2015**
(21) Application number: 06787104.6
(22) Date of filing: 12.07.2006
(51) Int. Cl.: A61B 18/00, A61B 18/14, A61B 1/05

(54) **MEDICAL-TREATMENT ELECTRODE ASSEMBLY AND METHOD FOR MEDICAL TREATMENT**
MEDIZINISCHE BEHANDLUNGSELEKTRODENANORDNUNG UND MEDIZINISCHES BEHANDLUNGSVERFAHREN
DISPOSITIFS D'ELECTRODES DE TRAITEMENT MEDICAL ET METHODE DE TRAITEMENT CORRESPONDANT

(30) Priority: 14.07.2005 US 181251
(43) Date of publication of application: 09.04.2008
(73) Proprietor: Ethicon Endo-Surgery, Inc., Cincinnati, OH 45242 (US)
(72) Inventor: CROPPER, Michael, S., Edgewood, KY 41017 (US); WEIZMAN, Patrick, Libery Township, OH 45044 (US); FRANER, Paul, T., Cincinnati, OH 45233 (US); YATES, David, West Chester, OH 45069 (US); NORVELL, David, K., Monroe, OH 45050 (US); HUITEMA, Thomas, Cincinnati, OH 45240 (US)
(74) Representative: Tunstall, Christopher Stephen
(86) International application number: PCT/US2006/027154
(87) International publication number: WO 2007/011634

(56) References cited:
- EP-A1- 1 518 498
- US-A- 6 033 397
- US-A1- 2002 072 741
- US-A1- 2003 181 900
- US-A1- 2003 229 293
- US-B1- 6 394 949
- US-B2- 6 740 082

## Description

### Field of the Invention

The present invention is related generally to medical systems, and more particularly to a medical-treatment electrode assembly and to a method for medical treatment.

### Background of the invention

A medical-treatment electrode assembly is known wherein a tube having two electrodes is inserted into a patient's esophagus, wherein the two electrodes are operatively connected to a medical radio-frequency (RF) generator, and wherein the two electrodes are brought into contact with esophageal tissue to treat gastro-esophageal reflux disease and other diseases of mucosal tissue.

Still, scientists and engineers continue to seek improved medical-treatment electrode assemblies and methods for medical treatment.

US 2003/0181900 discloses a tissue ablation system which Includes a plurality of electrode pairs and a viewing window between each pair of adjacent electrodes. The electrodes can be energized in a predetermined sequence to ablate tissue around the circumference of a body lumen.

### Summary

The invention provides a medical-treatment electrode assembly according to claim 1. The dependent claims include optional features.

Several benefits and advantages are obtained from one or more of the expressions of one or more of the embodiments of the invention. In one application, having a medical-treatment electrode which is flexible provides more intimate contact between the electrode and patient tissue which reduces charring of patient tissue and which improves non-visual monitoring of tissue, treatment. In the same or a different application, being able to have a video camera of a flexible endoscope view patient tissue between two medical-treatment electrodes allows the user to visually monitor tissue treatment for patient tissue between the two medical-treatment electrodes. In one implementation, having a medical-treatment electrode body with a central lumen operatively connectable to a vacuum source and with an opening extending from the outer surface of the medical-treatment electrode body to the central lumen provides a vacuum to draw patient tissue into more intimate contact with the electrode. In one employment, having patient-tissue-apparatus apparatus for moving patient tissue into a sidewall opening of a tube supporting a medical-treatment electrode tightens patient tissue outside the tube against, and into substantially full contact with, the medical-treatment electrode.

### Brief Description of the Figures

FIGURE 1 is a schematic, longitudinal cutaway view of an embodiment of a medical instrument which includes a first embodiment of a medical-treatment electrode assembly of the invention and which includes a medical radio-frequency (RF) generator and an embodiment of a flexible endoscope;
FIGURE 2 is a view of a portion of the medical-treatment electrode assembly of Figure 1, taken along lines 2-2 in Figure 1;
FIGURE 3 is not in accordance with the present invention and is a schematic, top planar view of a second arrangement of a medical-treatment electrode assembly;
FIGURE 4 is not in accordance with the present invention and is a cross-sectional view of the medical-treatment electrode assembly of Figure 3 taken along lines 4-4 in Figure 3;
FIGURES 5-7 are not in accordance with the present invention and are schematic, radial cross-sectional views of a patient's esophagus and a third arrangement of a medical-treatment electrode assembly showing various stages of patient esophageal tissue being drawn into a tube opening and of patient esophageal tissue outside the tube being tightened against two medical-treatment electrodes;
FIGURE 8 is not in accordance with the present invention and is a schematic, longitudinal cross-sectional view of a portion of a patient's esophagus and a cutaway view of a fourth arrangement of a medical-treatment electrode assembly showing patient tissue being drawn in a tube opening by grasping forceps and showing patient tissue outside the tube being tightened against a medical-treatment electrode supported by the tube;
FIGURE 9 is a side elevational view of a corkscrew retractor; and
FIGURE 10 is a side elevational view of a hook retractor.

### Detailed Description

Before explaining several embodiments of the present invention in detail, it should be noted that each embodiment is not limited in its application or use to the details of construction and arrangement of parts and steps illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described embodiments, expressions of embodiments, examples, etc. can be combined with any one or more of the other following-described embodiments, expressions of embodiments, examples, etc.

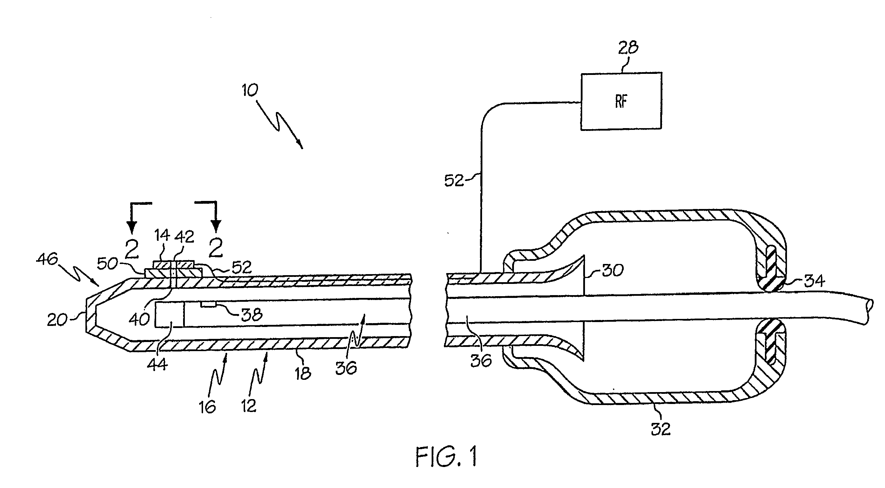

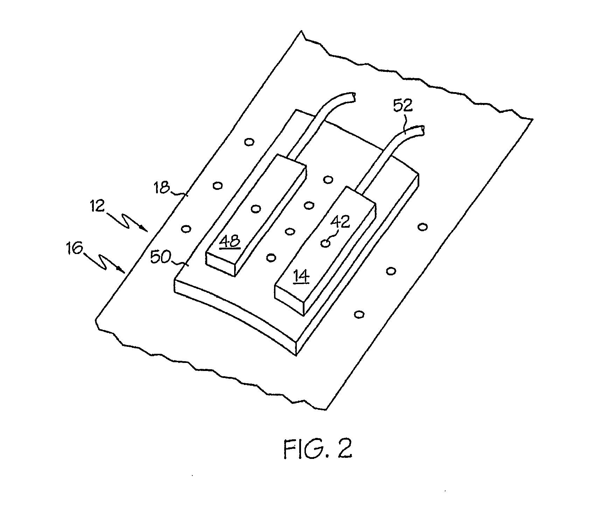

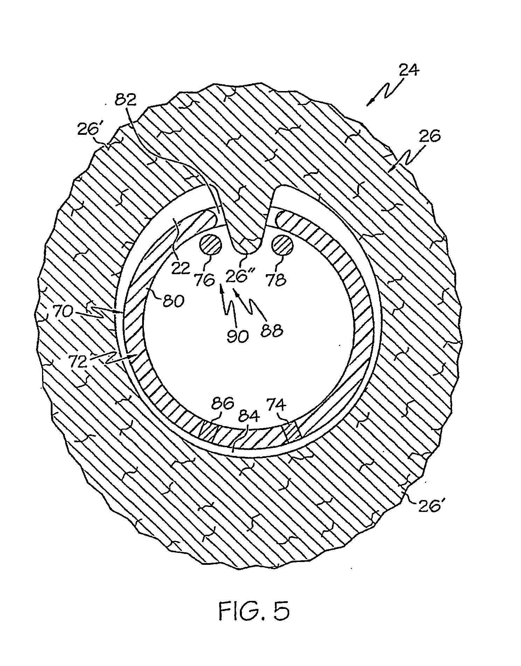

A first embodiment of a medical-treatment electrode assembly 10 of the invention is shown in Figures 1 and 2. A first expression of the embodiment of Figures 1 and 2 is for a medical-treatment electrode assembly 10 including a flexible tube 12 and a first (i.e., at least one) medical-treatment flexible electrode 14. The flexible tube 12 includes a sidewall 16 having an outer surface 18 and includes a distal end 20 insertable into a patient (such as, without limitation, insertable into the esophagus 22 of a patient 24 shown in Figure 5 for a third embodiment to be described later). The first medical-treatment flexible electrode 14 is fixedly supported (directly or indirectly) on the outer surface 18 proximate the distal end 20, is contactable with patient tissue (such as patient tissue 26 shown in Figure 5), and operatively connectable to a medical radio-frequency (RF) generator 28.

In one application, having a medical-treatment electrode which is flexible provides more intimate contact between the electrode and patient tissue which reduces charring of patient tissue and which improves non-visual monitoring of tissue treatment.

In one enablement of the first expression of the embodiment of Figures 1 and 2, the flexible tube 12 has a proximal end 30 disposable outside the patient 24. In this enablement, the medical-treatment electrode assembly 10 also includes a handle 32 surrounding, and attached to, the flexible tube 12 proximale the proximal end 30 and includes an annular seal 34 attached to the handle 32. The annular seal 34 is adopted to sealing receive a flexible endoscope 36 insertable into the flexible tube 12.

In one arrangement, wherein a flexible endoscope 36 is employed, the flexible endoscope 36 includes an aspiration port 38, and the sidewall 16 includes a through hole 40 disposed proximate the distal end 20 and in fluid communication with the aspiration port 38 of the flexible endoscope 36. In one variation, the flat medical-treatment flexible electrode 14 includes a through hole 42 aligned with the through hole 40 of the sidewall 16. In one application, providing a vacuum draws patient tissue into more intimate contact with the electrode.

In the some or a different arrangement, wherein a flexible endoscope 36 is employed, the flexible endoscope 36 includes a video camera 44, and the flexible tube 12 includes a distal end cap (e.g., 46) attached to the distal end of the sidewall 16. In one construction, the sidewall 16 is a monolithic sidewall extending from the distal end cap to the proximal end 30 of the flexible tube 12. In one variation, the distal end cap and the sidewall 16 are portions of a monolithic flexible tube 12. In one example, the distal end cap is chosen from the group consisting of a flexible tapered closed end cap 46, an open end cap (not shown) adapted to allow passage therethrough of the video camera of the flexible endoscope, and an end cap (not shown) adapted to open to allow passage therethrough of the video camera of the flexible endoscope and to close upon removal of the flexible endoscope therefrom.

In one employment of the first expression of the embodiment of Figure 1 and 2, a lead 52 operatively connects the first medical-treatmmt flexible electrode 14 to the radio-frequency (RF) generator 28. In one illustration, the lead 52 extends through a longitudinal channel in the sidewall 16 of the flexible tube 12. In one variation, the lead 52 from the electrode exits the flexible tube 12 through the sidewall 16 before reaching the handle 32. In another variation, not shown, the lead from the electrode exits the flexible tube 12 inside the handle 32 and then exits the handle 32. In one modification, not shown, the lead is operatively connected to a control button on the handle to start and stop the medical treatment. The lead and substrate are manufactured using etched circuit technology, wherein the lead is substantially flat and comprises, consists essentially of, or consists of copper, and the substrate is substantially flat and comprises, consists essentially of, or consists of polyester. In one deployment, shrink wrap, not down, surrounds the longitudinal juncture of the flexible tube and the distal end of the handle.

In one extension of the first expression of the embodiment of Figures 1 and 2, the medical-treatment electrode assembly 10 also includes a second medical-treatment flexible electrode 48. The second medical-treatment flexible electrode 48 is supported (directly or indirectly) on the outer surface 18 proximate the distal end 20 and is spaced apart from the first medical-treatment flexible electrode 14. The second medical-treatment flexible electrode 48 is contactable with the patient tissue 26 and is operatively connectable to the medical radio-frequency (RF) generator 28. It is noted that when only a single electrode is present, the assembly is operated as a monopolar assembly, and when two (or more) electrodes are present, the assembly can be operated as a monopolar and/or a bipolar assembly as can be appreciated by the artisan.

A second expression of the embodiment of Figures 1 and 2 is for a medical-treatment electrode assembly 10 including a flexible tube 12 and two medical-treatment (flexible or rigid) electrodes 14 and 48. The flexible tube 12 has an outer surface 18 and has a distal end 20 insertable into a patient 24. The two medical-treatment electrodes 14 and 48 are supported (directly or indirectly) on the outer surface 18 proximate the distal end 20, are contactable with patient tissue 26, and are operatively connectable to a medical radio-frequency (RF) generator 28. The two medical-treatment electrodes 14 and 48 are spaced apart, and a video camera 44 of a flexible endoscope 36 inserted into the flexible tube 12 and translated proximate the distal end 20 can view the patient tissue 26 between the two medical-treatment electrodes 14 and 48.

In one application, being able to have a video camera of a flexible endoscope view patient tissue between two medical-treatment electrodes allows the user to visually monitor tissue treatment for patient tissue between the two medical-treatment electrodes.

It is noted that the enablements, arrangements, variations, etc. of the previously-described first expression of the embodiment of Figures 1 and 2 are equally applicable to the second expression of the embodiment of Figures 1 and 2.

In one construction of the second expression of the embodiment of Figures 1 and 2, the flexible tube 12 is chosen from the group consisting of a transparent tube (as shown), a tube (not shown) having a solid transparent window disposed between the two medical-treatment electrodes, and a tube (not shown) having a tube cutout disposed between the two medical-treatment electrodes. In one example, when the flexible tube 12 is a transparent tube, the flexible tube 12 comprises, consists essentially of, or consists of polyethylene, polyurethane, or polyester. In one variation, the medical-treatment electrode assembly 10 also includes a transparent substrate 50 bonded to the outer surface 18 of the flexible tube 12, wherein the two medical-treatment electrodes 14 and 48 are bonded to the substrate 50. In one modification, the lead 52 is bonded to the substrate 50, and the substrate 50 (with the bonded lead 52) extends (not shown) on the outer surface 18 to proximate the proximal end 30 of the flexible tube 12. In one example, the substrate 50 comprises, consists essentially of, or consists of polyester. It is noted that additional unmarked through holes (similar to through holes 40 and 42) are shown as small circles on the second medical-treatment electrode 48, on the substrate 50, and on the flexible tube 12 in Figure 2. The number and layout of the through holes are left to the artisan.

A third expression of the embodiment of Figures 1 and 2 is for a medical-treatment electrode assembly including a flexible tube 12 and two medical-treatment flexible electrodes 14 and 48. The flexible tube 12 has an outer surface 18 and has a distal end 20 insertable into a patient 24. The two medical-treatment flexible electrodes 14 and 48 are fixedly supported on the outer surface 18 proximate the distal end 20, are contactable with patient tissue 26, and are operatively connectable to a medical radio-frequency (RF) generator 28. The two medical-treatment flexible electrodes 14 and 48 are spaced apart. A video camera 44 of a flexible endoscope 36 inserted into the flexible tube 12 and translated proximate the distal end 20 can view the patient tissue 26 between the two medical-treatment flexible electrodes 14 and 48.

It is noted that the enablements, arrangements, variations, constructions, etc. of the previously-described first and/or second expressions of the embodiment of Figures 1 and 2 are equally applicable to the third expression of the embodiment of Figures 1 and 2.

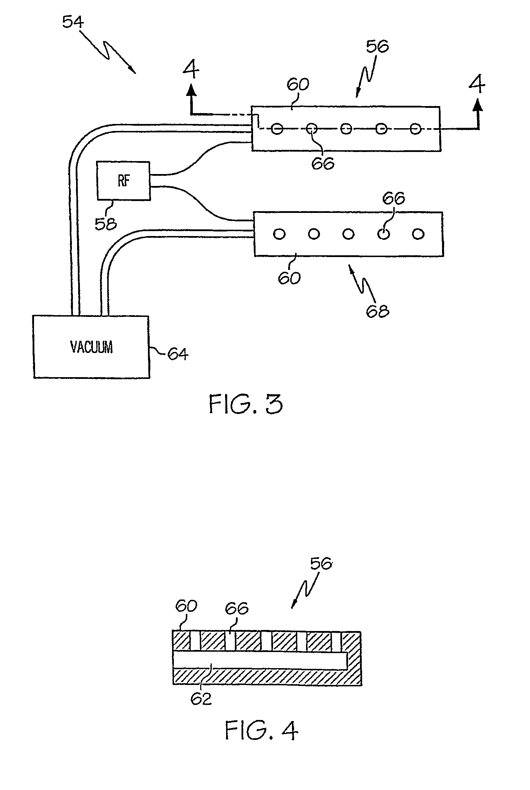

A second arrangement of a medical-treatment electrode assembly 54 is shown in Figures 3 and 4. A first expression of the arrangement of Figures 3 and 4 is for a medical-treatment electrode assembly 54 including a first medical-treatment electrode body 56 which is insertable into a patient 24. The first medical-treatment electrode body 56 is operatively connectable to a medical radio-frequency (RF) generator 58 and has an outer surface 60 contactable with patient tissue 26. The first medical-treatment electrode body 56 has a central lumen 62 operatively connectable to a vacuum source 64 and has an opening 66 extending from the outer surface 60 to the central lumen 62.

In one application, providing a vacuum draws patient tissue into more intimate contact with the electrode. Examples of vacuum sources 64 include, without limitation, syringes, squeeze bulbs, and pump motors.

In one extension of the first expression of the arrangement of Figures 3 and 4, the medical-treatment electrode assembly 54 also includes a second medical-treatment electrode body 68 insertable into the patient 24 and spaced apart from the first medical-treatment electrode body 56. The second medical-treatment electrode body 68 is operatively connectable to the medical radio-frequency (RF) generator 58 and has an outer surface 60 contactable with patient tissue 26. The second medical-treatment electrode body 60 has a central lumen (similar to the central lumen 62 of the first medical-treatment electrode body 56) operatively connectable to the vacuum source 64 and has an opening 66 extending from the outer surface 60 of the second medical-treatment electrode body 68 to the central lumen of the second medical-treatment electrode body 68.

In one application, the first and second medical-treatment electrode bodies 56 and 68 are supported on the outside of a flexible tube (not shown) in a manner similar to that shown for the embodiment of Figures 1 and 2.

A third arrangement of a medical-treatment electrode assembly 70 is shown in Figures 5-7. A first expression of the arrangement of Figures 5-7 is for a medical-treatment electrode assembly 70 including a flexible tube 72 (shown in cross-section), a medical-treatment electrode 74, and two rollers 76 and 78. The flexible tube 72 is insertable into a patient 24 and includes a sidewall 80 having an opening 82. The medical-treatment electrode 74 is supported by the flexible tube 72 and is contactable with patient tissue 26' which is outside the flexible tube 72. The medical-treatment electrode 74 is operatively connectable to a medical radio-frequency (RF) generator (such as RF generator 58 of Figure 3), and the flexible tube 72 is operatively connectable to a vacuum source (such as vacuum source 64 of Figure 3) to draw patient tissue 26" into the opening 82. The two rollers 76 and 78 are disposed inside the flexible tube 72 and are adapted to rollingly engage patient tissue 26", drawn into the opening 82 by the vacuum source, to draw more patient tissue 26" into the flexible tube 72 through the opening 82 to tighten patient tissue 26' outside the flexible tube 72 against the medical-treatment electrode 74.

In one configuration of the first expression of the arrangement of Figures 5-7, the opening 82 is located closer to the two rollers 76 and 78 than to the medical-treatment electrode 74. In the same or a different configuration, the two rollers 76 and 78 are translatable toward and away from each other. Mechanisms for rotating and translating the two rollers 76 and 78 are left to the artisan.

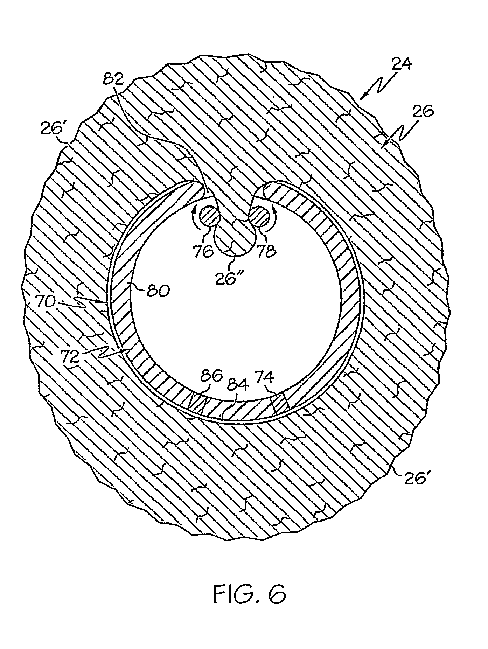

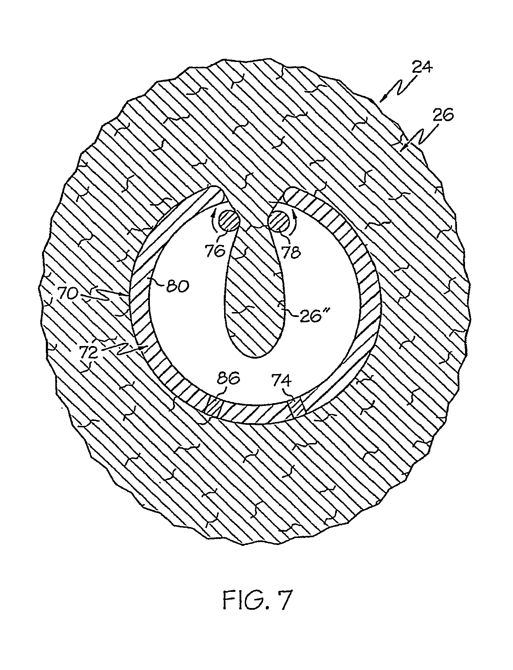

In one procedure employing the first expression of the arrangement of Figures 5-7, the flexible tube 72 of the medical-treatment electrode assembly 70 is inserted into the esophagus 22 of a patient 24 to have a few cellular layers of patient tissue 26" outside the flexible tube 72 be medically treated by the medical-treatment electrode 74. In one example, as shown in Figure 5, at first some patient tissue 26" is drawn through the opening 82 and into the flexible tube 72 by vacuum alone with the two rollers 76 and 78 spaced apart enough so as not to contact such patient tissue 26". In this example, as shown in Figure 6, then the two rollers 76 and 78 are brought toward each other to rollingly engage such patient tissue 26" to begin to draw more patient tissue 26" through the opening 82 and into the flexible tube 72. This action increases the tightening around the flexible tube 72 of patient tissue 26' outside the flexible tube 72. In this example, as shown in Figure 7, the two rollers 76 and 78 have drawn enough patient tissue 26" through the opening 82 and into the flexible tube 72 to cause patient tissue 26' outside the flexible tube 72 to more intimately contact the medical-treatment electrode 74. Notice how the gap 84 between the outside of the flexible tube 72 and the patient tissue 26' outside the flexible tube 72 decreases from Figure 5 to Figure 6 to Figure 7, and that the gap 84 disappears in Figure 7 between the patient tissue 26' outside the flexible tube 72 and the outside of the flexible tube 72 at the medical-treatment electrode 74.

It is noted that, in the example, the direction of rotation of the two rollers 76 and 78 to draw more patient tissue 26" through the opening 82 and into the flexible tube 72 is shown by unmarked arrows in Figures 6 and 7. In one construction, the surface of the two rollers 76 and 78 is textured to better grip patient tissue 26".

A second expression of the arrangement of Figures 5-7 is for a medical-treatment electrode assembly 70 including a flexible tube 72, two medical-treatment electrodes 74 and 86, and two rollers 76 and 78. The flexible tube 72 is insertable into a patient 24 and includes a sidewall 80 having an opening 82. The two medical-treatment electrodes 74 and 86 are supported by the flexible tube 72, are spaced apart, and are contactable with patient tissue 26' which is outside the flexible tube 72. The two medical-treatment electrodes 74 and 86 are operatively connectable to a medical radio-frequency (RF) generator (such as RF generator 58 of Figure 3), and the flexible tube 72 is operatively connectable to a vacuum source (such as vacuum source 64 of Figure 3) to draw patient tissue 26" into the opening 82. The two rollers 76 and 78 are disposed inside the flexible tube 72 and are adapted to rollingly engage patient tissue 26", drawn into the opening 82 by the vacuum source, to draw more patient tissue 26" into the flexible tube 72 through the opening 82 to tighten patient tissue 26' outside the flexible tube 72 against the two medical-treatment electrodes 74 and 86.

In one configuration of the second expression of the arrangement of Figures 5-7, the opening 82 is located closer to the two rollers 76 and 78 than to the two medical-treatment electrodes 74 and 86. In the same or a different configuration, the two rollers 76 and 78 are translatable toward and away from each other.

In one deployment of the second expression of the arrangement of Figures 5-7, the two rollers 76 and 78 are adapted to rollingly engage esophageal patient tissue 26", and wherein the two medical-treatment electrodes 74 and 86 are contactable with esophageal patient tissue 26' which is outside the flexible tube 72.

A third expression of the arrangement of Figures 5-7 is for a medical-treatment electrode assembly 70 which includes a tube 72 and a medical-treatment electrode 74. The tube 72 is insertable into a patient 24 and includes a sidewall 80 having an opening 82. The medical-treatment electrode 74 is supported by the tube 72, is contactable with patient tissue 26' which is outside the tube 72, and is operatively connectable to a medical radio-frequency (RF) generator (such as RF generator 28 of Figure 1 or RF generator 58 of Figure 3). The medical-treatment electrode assembly 70 also includes means 88 for moving patient tissue 26" into the opening 82 to tighten patient tissue 26' outside the tube 72 against the medical-treatment electrode 74.

In one example, the patient-tissue-moving means 88 includes a vacuum source (such as vacuum source 64 shown in figure 3). In the same or a different example, the patient-tissue-moving means 88 includes two rollers 76 and 78. In one variation, the means 88 includes only one of the vacuum source and the rollers. In a different variation, the means 88 includes both the vacuum source and the rollers. Other patient-tissue-moving means 88 includes a tissue grasper, a tissue pincher, a tissue pusher, tissue tweezers, and the like.

In one enablement, the patient-tissue-moving means 88 includes a tissue engaging device 90 (such as, but not limited to, rollers 76 and 78) adapted to mechanically pull and/or push patient tissue 26" into the opening 82 to tighten patient tissue 26' outside the tube 72 against the medical-treatment electrode 74. In one implementation, the tissue engaging device 90 is translatable and/or rotatable to mechanically pull and/or push patient tissue 26" into the opening 82 to tighten patient tissue 26' outside the tube 72 against the medical-treatment electrode 74. In one construction, the tissue engaging device 90 is disposed at the distal end of a flexible or rigid endoscope (not shown) which is insertable into the tube 72 of Figures 5-7 and which is operable from outside the patient.

Examples of tissue engaging devices 90 include, without limitation, the previously-described rollers 76 and 78, tissue gripping devices, tissue tweezing devices, tissue clamping devices, tissue squeezing devices, tissue reversibly penetrating devices, tissue holding devices, tissue pressing devices, and the like. Examples of tissue clamping devices include, without limitation, grasping forceps, forked jaw grasping forceps, rat tooth grasping forceps, three prong grasping forceps, tripod grasping forceps, fenestrated cup forceps, ellipsoid forceps, and the like. Examples of tissue reversibly penetrating devices include, without limitation, corkscrew retractors, hook retractors, and the like.

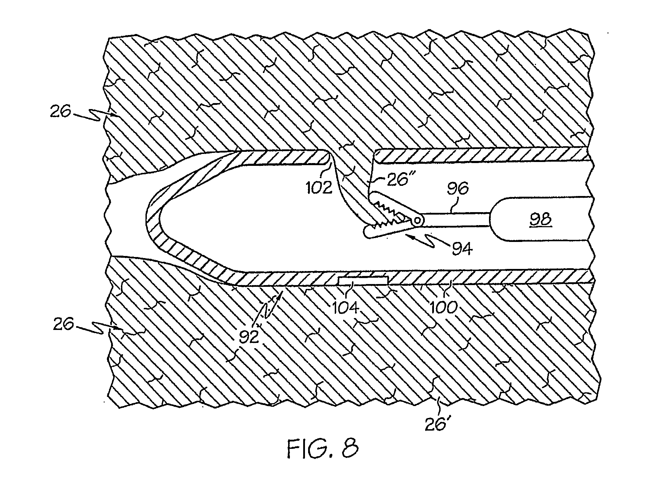

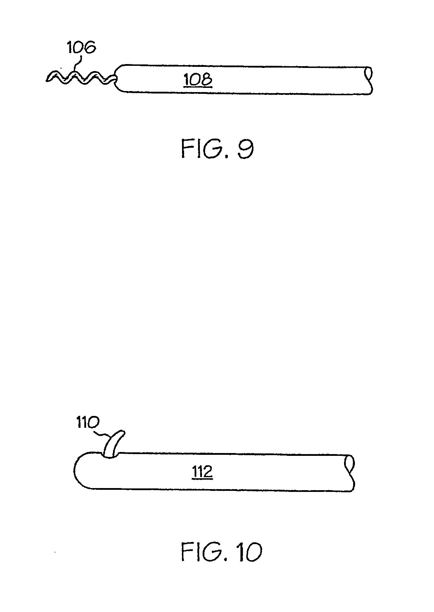

One arrangement of a medical-treatment electrode assembly 92 employing grasping forceps 94 is shown in Figure 8. The grasping forceps 94 are attached to the end of a translatable shaft 96 extending from the distal end of a flexible endoscope 98 disposed in a tube 100 having an opening 102. The grasping forceps 94 are shown pulling patient tissue 26" into the opening 102 to tighten patient tissue 26' outside the tube 100 against the medical-treatment electrode 104 which is supported by the tube 100. An embodiment of a corkscrew retractor 106 extending from the distal end of an endoscope 108 is shown in Figure 9, and an embodiment of a hook retractor 110 extending from a distal end portion of an endoscope 112 is shown in Figure 10. In one variation, the corkscrew retractor 106 and the hook retractor 110 are made of a shape memory alloy for easier insertion within the endoscope 108/112.

A first method not part of the invention described herein is for medical treatment and includes inserting a tube into a hollow body organ of a patient, wherein the tube supports a medical-treatment electrode and has a sidewall opening. The first method includes then moving patient tissue into the sidewall opening to tighten patient tissue outside the tube into substantially full contact with the medical-treatment electrode. The first method includes then activating the medical-treatment electrode.

A second method not part of the invention described herein is for medical treatment and includes inserting a tube into an abdominal or thoracic cavity of a patient, wherein the tube supports a medical-treatment electrode and has a sidewall opening. The second method includes then moving patient tissue into the sidewall opening to tighten patient tissue outside the tube into substantially full contact with the medical-treatment electrode. The second method includes then activating the medical-treatment electrode.

Several benefits and advantages are obtained from one or more of the expressions of one or more of the embodiments of the invention. In one application, having a medical-treatment electrode which is flexible provides more intimate contact between the electrode and patient tissue which reduces charring of pateint tissue and which improves non-visual monitoring of tissue treatment. In the same or a different application, being able to have a video camera of a flesrible endoscope view patient tissue between two medical-treatment electrodes allows the user to visually monitor tissue treatment for patient tissue between the two medical-treatment electrodes. In one implementation, having a medical-treatment electrode body with a central lumen operatively connectable to a vacuum source and with an opening extending from the outer surface of the medical-treatment electrode body to the central lumen provides a vacuum to draw patient tissue into more intimate contact with the electrode. In one employment, having pateint-tissue-moving apparatus for moving patient tissue into a sidewall opening of a tube supporting a medical-treatment electrode tightens patient tissue outside the tube against, and into substantially full contact with, the medical-treatment electrode.

## Claims

1. A medical-treatment electrode assembly (10) comprising:
a) a flexible tube (12) including a sidewall (16) having an outer surface (18) and including a distal end (20) insertable into a patient; and
b) a first medical-treatment flexible electrode (14) fixedly supported on the outer surface of the flexible tube (12) proximate the distal end, contactable with patient tissue, and operatively connectable to a medical radio-frequency (RF) generator (28);
wherein the sidewall includes a through hole (40) disposed proximate the distal end and arranged for fluid communication with an aspiration port (38) of a flexible endoscope (36);
wherein the first medical-treatment flexible electrode includes a through hole (42) aligned with the through hole of the sidewall.

2. The medical-treatment electrode assembly (10) of claim 1, also including a second medical-treatment flexible electrode (48) supported on the outer surface (18) proximate the distal end (20), spaced apart from the first medical-treatment flexible electrode (14), contactable with the patient tissue, and operatively connectable to the medical radio-frequency (RF) generator (28).

3. The medical-treatment electrode assembly (10) of claim 2, wherein a video camera (44) of the flexible endoscope (36) inserted into the flexible tube (12) and translated proximate the distal end (20) can view the patient tissue between the two medical-treatment electrodes (14, 48).

4. The medical-treatment electrode assembly (10) of claim 1 or 3, wherein the flexible tube (12) has a proximal end (30) disposable outside the patient, and also including a handle (32) surrounding and attached to the flexible tube proximate the proximal end and including an annular seal (34) attached to the handle, wherein the annular seal is adapted to receive the flexible endoscope (36) insertable into the flexible tube.

5. The medical-treatment electrode assembly (10) of claim 4, wherein the flexible tube (12) includes a distal end cap (46), and wherein the distal end cap is chosen from the group consisting of a flexible tapered closed end cap, an open end cap adapted to allow passage therethrough of the video camera (44) of the flexible endoscope (36), and an end cap adapted to open to allow passage therethrough of the video camera of the flexible endoscope and to close upon removal of the flexible endoscope therefrom.

## Patentansprüche

1. Medizinische Behandlungselektrodenanordnung (10), umfassend:
a) einen flexiblen Schlauch (12), der eine Seitenwand (16) mit einer Außenseite (18) und ein in einen Patienten einführbares distales Ende (20) aufweist; und
b) eine erste flexible medizinische Behandlungselektrode (14), die fest auf der Außenseite des flexiblen Schlauchs (12) in der Nähe des distalen Endes gestützt wird, mit Gewebe des Patienten in Berührung gebracht werden kann und mit einem medizinischen Radiofrequenz (RF)-Generator (28) in Wirkverbindung gebracht werden kann;
worin die Seitenwand ein Durchgangsloch (40) aufweist, das in der Nähe des distalen Endes angeordnet ist und zur Fluidverbindung mit einer Ansaugöffnung (38) eines flexiblen Endoskops (36) angeordnet ist;
worin die erste flexible medizinische Behandlungselektrode ein Durchgangsloch (42) aufweist, das mit dem Durchgangsloch der Seitenwand ausgerichtet ist.

2. Medizinische Behandlungselektrodenanordnung (10) nach Anspruch 1, die auch eine zweite flexible medizinische Behandlungselektrode (48) aufweist, die auf der Außenseite (18) in der Nähe des distalen Endes (20) gestützt wird, im Abstand von der ersten flexiblen medizinischen Behandlungselektrode (14) angeordnet ist, mit dem Gewebe des Patienten in Berührung gebracht werden kann und mit dem medizinischen Radiofrequenz (RF)-Generator (28) in Wirkverbindung gebracht werden kann.

3. Medizinische Behandlungselektrodenanordnung (10) nach Anspruch 2, worin eine Videokamera (44) des flexiblen Endoskops (36), die in den flexiblen Schlauch (12) eingeführt wird und in die Nähe des distalen Endes (20) geschoben wird, das Gewebe des Patienten zwischen den zwei medizinischen Behandlungselektroden (14, 48) betrachten kann.

4. Medizinische Behandlungselektrodenanordnung (10) nach Anspruch 1 oder 3, worin der flexible Schlauch (12) ein proximales Ende (30), das außerhalb des Patienten angeordnet werden kann, und auch einen Handgriff (32) aufweist, der den flexiblen Schlauch umgibt und in der Nähe des proximalen Endes an dem flexiblen Schlauch befestigt ist, und eine an dem Handgriff befestigte ringförmige Dichtung (34) aufweist, worin die ringförmige Dichtung zur Aufnahme des in den flexiblen Schlauch einführbaren flexiblen Endoskops (36) ausgelegt ist.

5. Medizinische Behandlungselektrodenanordnung (10) nach Anspruch 4, worin der flexible Schlauch (12) eine distale Endkappe (46) aufweist, und worin die distale Endkappe aus der aus einer flexiblen verjüngten geschlossenen Endkappe, einer offenen Endkappe, die ausgelegt ist, die Durchführung der Videokamera (44) des flexiblen Endoskops (36) durch sie hindurch zu gestatten, und einer Endkappe, die geöffnet werden kann, um die Durchführung der Videokamera des flexiblen Endoskops durch sie hindurch zu gestatten, und die nach Entfernung des flexiblen Endoskops daraus geschlossen werden kann, bestehenden Gruppe ausgewählt ist.

## Revendications

1. Dispositif d'électrodes de traitement médical (10) comprenant :
a) un tuyau flexible (12) comprenant une paroi latérale (16) ayant une surface externe (18) et comprenant une extrémité distale (20) insérable dans un patient ; et
b) une première électrode flexible de traitement médical (14) supportée de manière fixe sur la surface externe du tuyau flexible (12) à proximité de l'extrémité distale, pouvant être placée au contact du tissu d'un patient, et pouvant être opérationnellement connectée à un générateur radiofréquence (RF) médical (28) ;
où la paroi latérale comprend un trou traversant (40) disposé à proximité de l'extrémité distale et conçu pour une communication fluide avec un port d'aspiration (38) d'un endoscope flexible (36) ;
où la première électrode flexible de traitement médical comprend un trou traversant (42) aligné avec le trou traversant de la paroi latérale.

2. Dispositif d'électrodes de traitement médical (10) selon la revendication 1, comprenant en outre une seconde électrode flexible de traitement médical (48) supportée sur la surface externe (18) à proximité de l'extrémité distale (20), espacée de la première électrode flexible de traitement médical (14), pouvant être placée au contact du tissu d'un patient, et pouvant être opérationnellement connectée au générateur radiofréquence (RF) médical (28).

3. Dispositif d'électrodes de traitement médical (10) selon la revendication 2, dans lequel une caméra vidéo (44) de l'endoscope flexible (36) inséré dans le tuyau flexible (12) et translaté à proximité de l'extrémité distale (20) peut voir le tissu du patient entre les deux électrodes de traitement médical (14, 48).

4. Dispositif d'électrodes de traitement médical (10) selon la revendication 1 ou la revendication 3, dans lequel le tuyau flexible (12) a une extrémité proximale (30) disponible à l'extérieur du patient, et comprenant également une poignée (32) entourant et fixée au tuyau flexible à proximité de l'extrémité proximale et comprenant un joint annulaire (34) fixé à la poignée, où le joint annulaire est conçu pour recevoir l'endoscope flexible (36) insérable dans le tuyau flexible.

5. Dispositif d'électrodes de traitement médical (10) selon la revendication 4, dans lequel le tuyau flexible (12) comprend un capuchon d'extrémité distale (46), et où le capuchon d'extrémité distale est choisi dans le groupe constitué d'un capuchon d'extrémité fermé conique, d'un capuchon d'extrémité ouvert conçu pour permettre d'y passer à travers la caméra vidéo (44) de l'endoscope flexible (36) et d'un capuchon d'extrémité conçu pour s'ouvrir pour permettre d'y passer à travers la caméra vidéo de l'endoscope flexible et pour se refermer lors du retrait de l'endoscope flexible de celui-ci.
